# EUROPEAN PATENT APPLICATION

(11) **EP 3 093 016 A1**
(43) Date of publication of application: **16.11.2016**
(21) Application number: 16169524.2
(22) Date of filing: 13.05.2016
(51) Int. Cl.: A61K 31/404, A61K 36/31, A61K 36/48, A61K 36/704, A61P 17/06

(54) **COMPOSITIONS FOR THE TREATMENT OF PSORIASIS**

(30) Priority: 14.05.2015 IT RM20150208
(71) Applicant: DIEFFETTI Cosmetici S.r.l. semplificata, 76013 Minervino Murge (BT) (IT)
(72) Inventor: DI SALVO, Gaetano, 76013 Minervino Murge BT (IT)
(74) Representative: Di Giovine, Paolo

(57) **Abstract**

The present invention relates to compositions comprising isolated indirubin for topical use in the treatment of psoriasis or of other dermatitides caused by inflammatory processes.

## Description

The present invention relates to compositions comprising isolated indirubin for topical use in the treatment of psoriasis or of dermatitides caused by inflammatory processes.

### PRIOR ART

Psoriasis is a chronic skin disease of inflammatory nature that can manifest itself in all body areas in different forms: from deep purple red areas to thickened areas with white edges. The various forms of psoriasis are commonly classified as follows:
- Plaque psoriasis;
- Guttate psoriasis;
- Inverse psoriasis;
- Erythrodermic psoriasis;
- Pustular psoriasis;

The most widespread form is the plaque one, with an incidence of about 80%. The most common manifestations are well-delimited erythematous papules and plaques covered with silvery or opalescent scales. Lesions are of various sizes, and severity can vary from a few spots of dandruff-like desquamation to general dermatosis with debilitating arthritis (psoriatic arthritis), exfoliations and eruptions. In spite of its name meaning "itchy condition", itch is not always present; in some cases, said symptom is present in psoriatic vulvites, where lesions from scratching can be similar to those from *lichen simplex.* The most common sites for lesions are the scalp (including the retroauricular zone), elbow and knee extension zones and the lumbosacral zone, but in some of its forms it is found in flexion zones, on genitals and on foot soles and hand palms. The lesions heal without leaving scars and without perturbing hair growth. 30-50% of subjects exhibits nail degeneration, with hyperkeratosis, thickening, subungual detritus, onicholysis, distortion. In the eruptive phase, a trauma can cause the appearance of linear lesions (Koebner phenomenon). Psoriasis has a markedly negative impact on the life of whom is affected thereby. Severe psoriasis cases have repercussions on a patient's health and quality of life to an extent similar to other chronic diseases, such as, e.g., depression, arterial hypertension, congestive heart failure, diabetes mellitus, etc. Depending on lesion severity and localization, subjects affected by psoriasis may experience marked physical discomfort and some disability. Itch and pain can interfere with many normal daily activities, like, e.g., personal care or sleep. The presence of patches on the hands or feet can prevent the patient from practicing certain professions, some sporting activities (for instance, swimming), and sometimes even the mere relating to other people. Scalp-localized patches can be particularly embarrassing, and desquamating plaques in hair are often mistaken for dandruff.

Some patients radically change their social habits due to the disease. Feeling restricted in going to the beach on the seaside, or being unable to go to a swimming pool, dreading to wear short or sleeveless clothing on certain occasions, lest the patches be in view, feeling the need to cover oneself before meeting someone for the first time, for fear of not knowing which reactions he/she might feel due to psoriasis - all this is decidedly frustrating and limiting. The feeling of discomfort and the social impact are certainly more severe and serious for younger or teenage patients. A teenager unable to take part in all activities of his/her peer group tends to go through frustration, shame and rage. Not everybody succeeds at developing adequate strategies for living with the disease. Psychological discomfort can lead to significant depression, and to social withdrawal and isolation. Young people, as well as adults, develop a poor image of themselves which stems from worries related to dread of public rejection, and to worries inherent to the emotional and sexual sphere. A research conducted by the European Federation of Psoriasis Patient Associations (EUROPSO) highlighted that more than half of the patients affected by severe psoriasis considers his/her condition a significant problem in carrying on everyday life.

There are many instruments for measuring the quality of life of patients affected by psoriasis and other dermatological disorders. Some scientific studies have confirmed that patients often experience a decrease in the quality of life. Among the symptoms, above all itch seems to contribute to a greater extent to deteriorating the quality of life. A 2010 study has verified the effectiveness of a simple itch self-evaluation scale, subdivided into 6 points (0 = no itch; 5 = highly severe itch) or (itch), and based on patients' responses. The conclusions of the study are favourable to the use of this self-evaluation tool, deeming it a valid measure of itch intensity in patients with moderate to severe plaque psoriasis. The scale would also be able to discriminate between subjects in need of a specific treatment and those not in need thereof. Current therapies for the treatment of psoriasis are very expensive, often are not decisive or entail various side effects, e.g. the harmful effects due to use of cortisone preparations are known.

Patent EP2489358 describes an oil-extracted product comprising indirubin, obtained by heating *indigo naturalis* powder, and its use in the treatment of psoriasis.

Therefore, object of the present invention is to provide new compositions for topical use in the treatment of psoriasis or of other dermatitides caused by inflammatory processes.

### SUMMARY OF THE INVENTION

It was surprisingly discovered that the topical application of compositions comprising as active principle isolated indirubin is an effective cure in the treatment of psoriasis and of other dermatitides caused by inflammatory processes, entailing further advantages and a greater effectiveness with respect to compositions described in the state of the art. Therefore, object of the present invention are compositions comprising as active principle isolated indirubin for topical use in the treatment of psoriasis or of other dermatitides caused by inflammatory processes. The treatment of the present invention is of extremely easy application and can be carried out without the intervention of any specific professional figure and, also, the improvements induced are as easy to notice, without a medical consultation being needed.

Still further advantages, as well as the features and the modes of employ of the present invention will be made apparent in the following detailed description of some preferred embodiments thereof, given by way of example and not for limitative purposes. Reference will be made to the figures of the annexed drawings, particularly in the light of the comparative experiments described in the experimental section.

### DETAILED DESCRIPTION OF THE FIGURES

Fig. 1. In Figure 1 is reported the photo of a foot of a patient affected by psoriasis with a high degree of severity of the conditions.
Fig. 2. In Figure 2 is reported the photo of the same foot of Figure 1 after treatment with the composition of the present invention; as is evident from the photo, the degree of severity of the disease after treatment is extremely improved.
Fig. 3. In Figure 3 is reported the photo of an arm of a patient affected by a severe form of psoriasis, before and after a 30-day treatment with the composition of the present invention, with one application twice per day.
Fig. 4. In Figure 4 is reported the photo of a foot of a patient affected by a severe form of psoriasis before and after a treatment of only 15 days with the composition of the present invention, with one application twice per day.

### DETAILED DESCRIPTION OF THE INVENTION

The present invention refers to compositions for use in the treatment of psoriasis or of other dermatitides caused by inflammatory processes. Such a composition proved particularly effective in the cure, the prevention and the slowing down of the most severe manifestations of psoriasis, in particular in the treatment of the different types of psoriasis, such as plaque psoriasis, guttate psoriasis, inverse psoriasis, erythrodermic psoriasis.

Such a composition proved effective also in the treatment of other dermatitides caused by inflammatory processes, in particular in contact dermatitis. In the present description, by the term "dermatitis" it is meant a pathology where a skin inflammation subsists, caused, e.g., by an external agent.

By the term "isolated indirubin" it is meant that indirubin is used in the composition with a high degree of purity, e.g., 99%, 98%, 95%, 90%, or anyhow with a purity higher than the indirubin obtainable with the oil extraction processes described in EP2489358.

The composition could contain one or more among carriers, diluents, excipients, emulsifiers, emollients, preservatives, consistency factors, pH adjusters, antioxidants (like, e.g., vitamins, tocopherols or other antioxidants known in the field), suitable for the making of formulations for topical use (cosmetic formulations, pharmaceutical ones or medical devices) like, e.g., or more glycerol, xanthan gum, potassium sorbate, sodium silicate, cetearyl glucoside, cetearyl alcohol, cetyl stearyl alcohol, hydrogenated castor oil, glycerol monostearates, dicaprylyl carbonate, citric acid, stearic acid, dimethicone, triethanolamine, urea, glyceryl oleate, carbomer, in particular coconut oil, hemp oil and/or beeswax.

Such compositions could be prepared according to the techniques known to the technician in the field by utilizing the above-described compounds, e.g. by mixing the individual compounds directly during the preparing of the composition, or by adding to the carriers and/or diluents and/or excipients a mixture of the compounds previously prepared.

The composition according to the present invention could be made in the form of cosmetic, pharmaceutical composition, or of medical device according to any one of the classes described in Directive 93/42/EEC on medical devices (comprising also substances and not only "devices" in the mechanical sense of the term), or in any suitable form according to the regulatory provisions of the Country in which said composition will be produced.

When made in the form of a cosmetic or pharmaceutical composition, the components utilized, as well as the excipients, will be of a grade cosmetically or pharmaceutically acceptable for the desired use (topical, in this case). Such grade could be utilized also for the components and excipients for the preparing of the composition as a medical device or of the composition comprised in a medical device.

In one embodiment, the composition will be formulated to be suitable to be administered on the scalp, therefore it will not be olive oil-based, but it will comprise, e.g., Coco-Caprilate, isolated Indirubine, and optionally one or more perfumed essences.

In one embodiment, it could further comprise Coco-Caprilate and/or Castor oil and/or Vaseline and/or Beeswax and/or Capric Triglyceride.

In one embodiment, the present invention could be a dressing for dermatological use like, e.g., a patch, a gauze or a bandage to be applied on the concerned area comprising the compositions according to the present invention. E.g., a gauze soaked of a pomade, ointment or cream described herein.

According to a non-limiting example, the composition of the present invention could be administered once or more per day by local (topical) application on the parts to be treated for a time period, e.g., of 1, 2, 3, 4 weeks, or even longer. According to one embodiment, it will be administered at least once per day, for at least one week. The administration regimens could vary on the basis of disease severity.

The compositions for topical use according to the present invention could be in any one form selected from gel, ointment, cream, pomade, foam, powder, spray, oil-in-water emulsion, water-in-oil emulsion, oil-in-gel emulsion, gel-in-oil emulsion, suspension, paste.

The ointment is defined as a composition comprised of a monophasic base in which solid or liquid substances can be dispersed. According to one embodiment, the compositions could be hydrophobic (lipophilic) ointments: i.e., they can absorb only small amounts of water. Typical substances used for their preparing are solid, semisolid and liquid paraffins, plant oils, animal fats, synthetic glycerides, waxes and liquid polyalkyl oxylanes. According to another embodiment, said compositions could be water-emulsioning ointments: they can absorb greater amounts of water and therefore form water-in-oil (W/O) emulsions or oil-in-water (O/W) emulsions according to the emulsion nature: for this purpose, W/O surfactants could be used, such as wool alcohols, sorbitan esters, monoglycerides and fatty alcohols, or O/W emulsifiers such as fatty alcohols sulfates, polysorbates, macrogol cetostearyl esters or ester of fatty acids with macrogols. Their bases are those of hydrophobic ointments. Hydrophilic ointments are instead preparations having water-miscible bases. The bases are, for instance, mixtures of liquid and solid macrogols (polyethylene glycols). They can contain appropriate amounts of water.

Creams are multiphase preparations comprised of a lipophilic phase and an aqueous phase. Hydrophobic creams have the lipophilic phase as continuous phase. They contain water-in-oil (W/O) emulsifiers, such as wool alcohols, sorbitan esters and monoglycerides. Hydrophilic creams have the hydrophilic phase as continuous phase. They contain oil-in-water (O/W) emulsifiers, such as sodium and triethanolamine soaps, fatty alcohol sulfates, polysorbates and polyhydroxylated fatty acids and esters of fatty acids associated, if necessary, with water-in-oil emulsifiers.

Gels are comprised of liquids gelled with suitable gelling agents. Hydrophobic gels (oleogels) are preparations whose bases are usually comprised of liquid paraffin, with polyethylene or fatty oil gelled with colloidal silica or soaps of aluminium or zinc. Hydrophilic gels (hydrogels) are preparations whose bases usually contain water, glycerol or propylene glycol, gelled with appropriate substances such as starch, cellulose derivatives, carboxyvinyl polymers and magnesium aluminium silicates. Pastes instead are semisolid preparations for cutaneous applications which contain, finely dispersed in the base, solids in high proportions.

The compositions of the present invention comprise indirubin (CAS number 479-41-4), a substance that can be extracted both from leaves and dry pigment of various plants, among which *Indigofera tinctoria, Baphieacanthuscusia, Isatistinctoria, Polygonum tinctorium* ed *Isatis indigotica.* The extract of the above-mentioned plants is known in the state of the art with the name of *Indigo naturalis.*

Indirubin can be obtained by various processes known in the state of the art, both by extraction methods with solvents from the above-mentioned natural sources and by chemical synthesis processes; these processes were developed from the mid-19th century, until reaching syntheses reworked and developed in the last years. Moreover, indirubin can be purchased from several providers of raw materials for chemical processes.

According to a preferred embodiment, the composition will comprise indirubin obtained from *Indigofera tinctoria, Baphieacanthuscusia, Isatistinctoria, Polygonum tinctorium* and *Isatis indigotica* plants. Indirubin will be purified from other substances contained in the extracts of those plants. The compositions will therefore be formulated so as not to contain other substances comprised in the extracts, e.g. in the *Indigo naturalis* extract. According to one embodiment, indirubin is in a concentration between 0.1 and 10 mg per each gram of composition. Experimentation highlighted that also dosages lower than 1 mg per gram of composition are effective in nearly 75% of cases analysed. In the remaining 25%, encompassing clinical cases with a higher degree of disease, a higher dosage is needed, yet always falling within the above-indicated range. According to one embodiment, indirubin will therefore be present in the composition in a concentration between 1 and 10 mg per each gram of composition, e.g. between 5.00 and 10.00 mg per each gram of composition.

Examples aimed at better illustrating the present invention, and some specific embodiments, are reported below; such examples are in no way to be considered as a limitation of the preceding description and of the subsequent claims.

### EXAMPLES

### COMPOSITION EXAMPLES

The composition according to the present invention could be prepared by mixing, by conventional techniques, the active components according to the detailed description and optional additional components as above-indicated, for instance appropriate agents suitable for preparing compositions for topical use, like, e.g., carriers, diluents, excipients, emulsifiers, emollients, preservatives, disaggregants, antioxidants. No specific preparation protocols are needed, since conventional protocols are suitable for making the composition in any form indicated in the present description. The preparation examples are not limitative of the present description, but serve to exemplify some of the possible embodiments thereof.

**COMPOSITION EXAMPLE 1 (amount for 100 g of product)**

| INGREDIENT | AMOUNT GRAMS |
|---|---|
| Indirubin | 0.1 |
| Beeswax | 12 |
| Coconut oil | 15 |
| Cocoa butter | 18 |
| Karite butter | 18 |
| Perfume | 0.5 |
| Olive oil | q.s. to 100 g |
| | |

**COMPOSITION EXAMPLE 2 (amount for 100 g of product)**

| INGREDIENT | AMOUNT GRAMS |
|---|---|
| Indirubin | 0.25 |
| Hemp oil | 25 |
| Cetearyl Alcohol | 5 |
| Glycerol | 3 |
| D-Panthenol | 2 |
| Emulsifier | 5 |
| Perfume | 0.5 |
| Water | q.s. to 100 g |

**COMPOSITION EXAMPLE 3 (amount for 100 g of product)**

| INGREDIENT | AMOUNT GRAMS |
|---|---|
| Indirubin | 0.5 |
| Hemp oil | 25 |
| Cetearyl Alcohol | 5 |
| Glycerol | 3 |
| D-Panthenol | 2 |
| Emulsifier | 5 |
| Perfume | 0.5 |
| Water | q.s. to 100 g |

**COMPOSITION EXAMPLE 4 (amount for 100g of product)**

| INGREDIENT | AMOUNT GRAMS |
|---|---|
| Indirubin | 0.75 |
| Coco-Caprilate | 80 |
| Perfume | 0.5 |
| Castor oil | q.s. to 100 g |

**COMPOSITION EXAMPLE 5 (amount for 100g of product)**

| INGREDIENT | AMOUNT GRAMS |
|---|---|
| Indirubin | 1 |
| Strinqy petroleum jelly | 80 |
| Beeswax | 5 |
| Perfume | 0.5 |
| Caprilic/Capric Triglyceride | q.s. to 100 g |

### PREPARATION OF THE COMPOSITION OF EXAMPLE 1

### COMPOSITION 1

In a vessel of suitable size, all components except perfume are introduced. The whole is brought to complete melting, then slowly stirred to amalgamate it. Indirubin can be added at this point, or it can be previously hot-dissolved in oil, or can derive from the extraction.

Start cooling, and add perfume when the mixture is still markedly fluid. Then, meter the fluid mixture in its container and wait for the whole to reach room temperature.

### COMPOSITION 2

In a suitable vessel, introduce all fatty phase components and bring the whole to 75°C. Indirubin can be added at this point, or it can be previously hot-dissolved in oil, or can derive from the extraction.

In another vessel, provided with turbo-stirring, introduce all aqueous phase components and bring the whole to a 75°C temperature as well.

At this point, introduce the fatty phase at 75°C into the vessel containing the aqueous phase at the same temperature and proceed to a vigorous stirring for several minutes with the turbo-stirrer, so as to generate the emulsion.

Then, start the slow cooling, keeping a constant and slow stirring in the vessel.

When the temperature will have reached 40°C, add perfume and D-Panthenol and continue with the cooling under stirring.

At room temperature, procedure with packing of the emulsion in the specially provided containers.

### EXPERIMENTAL RESULTS

8 informed subjects affected by psoriasis were recruited. For each patient, a photographic documentation of the zones concerned by psoriasis prior to the treatment was produced.

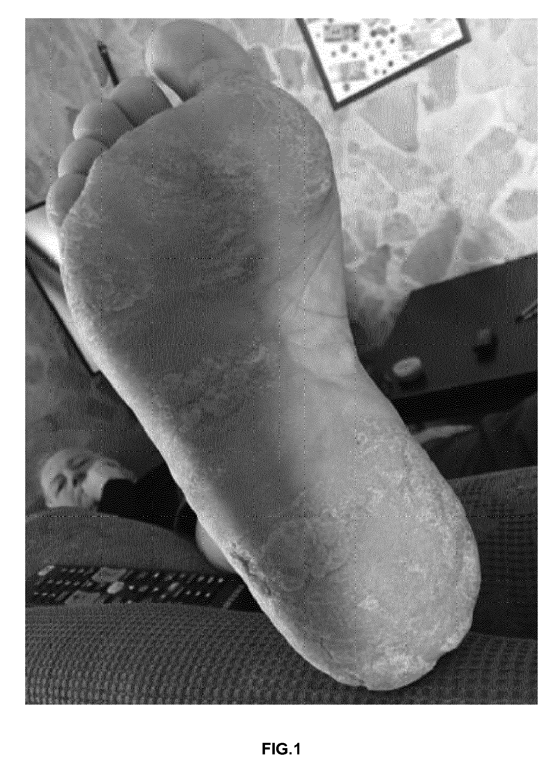

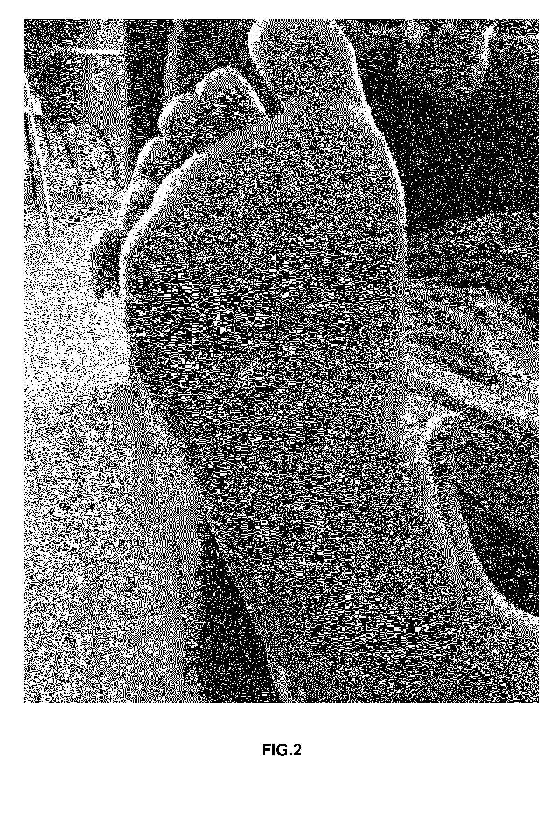

Various formulations containing different concentrations of indirubin, in the range of 1-10 mg Indirubin per gram of final formulation, were delivered to the patients. Each of the subjects used the assigned product depending on his/her normal daily life habits - someone thrice a day, somebody twice, somebody else once, due to a working activity that did not allow more frequent applications. Indications on treatment duration were of about 8 weeks; however, part of the subjects undergoing the treatment noticed a sensible improvement of psoriasis symptomatology, already from the first applications. The most sensational case is that of the subject reported in Figs. 1 and 2, for whom the reported result was attained in a mere 10 days.

From the experimentation carried out it can be concluded that:
The higher concentration and the greater frequency of application of the preparations causes a faster response in the improvement of symptomatology;
in 2 of the 8 subjects treated only a minimum improvement was detected, not comparable to the results reported in the photos.

The percentage of non-responders, i.e. of the subjects who did not benefit from the treatment, is in the neighbourhood of 25%, which anyhow would mean that 75% of subjects affected by psoriasis would instead receive a sensible benefit. Treated for 20 days with the composition of the invention as described in the example. At the end of the treatment the zones concerned by psoriasis were photographed again; the photos highlighted a marked improvement in the more severe cases, or, in less severe cases, a marked remission. By way of example, a case before and after treatment is shown, respectively in Figures 1 and 2.

### Comparative examples

Compared to the formulations described in EP2489358, obtained by oil extraction from *Indigo Naturalis,* the formulations according to the present invention based on the sole active principle INDIRUBIN entail numerous advantages.

The use of the isolated active principle Indirubin allow to obtain extremely varied formulations, both in composition, thereby allowing use in different applications, and in concentration, with the entailed possibility of modulating the dosage relative to the severity of manifestation and/or modulating the dosage relative to the obtainment rate of the final result. In more detail, the compositions described in the prior art, such as the oil-extracted product described in EP2489358, cannot provide a firm administration dosage, as there may be a marked variation in the titre, depending on the *Indigo Naturalis* that is used as starting material. Various experimental tests have indicated that the indirubin titre can vary sensibly, depending on origin and age (years) of the plant used for preparing the extract. Moreover, in some clinical cases, in order for the treatment to be effective it is necessary to use an indirubin dosage higher than the one obtainable with the oil extraction described in EP2489358.

Formulations based on the sole INDIRUBIN according to the present invention, with respect to those described in the prior art, are therefore quicker in obtaining the final result, as it is possible to formulate different types of products that may be used even where oil extraction-derived products cannot be used (or are aesthetically unacceptable) due to high oiliness, like, e.g., formulations for scalp treatment. Comparative experiments were conducted by treating in parallel patients with the extract described in Patent EP2489358 and with the composition according to the present invention, which uses isolated indirubin.

More specifically, the arms, legs and/or feet of various patients affected by severe forms of psoriasis were treated in parallel with the formulation of the present invention and with the composition described in EP2489358, obtained with the following conditions:
Extraction oil: Olive oil
Extraction temperature: 145°C
Extraction time: 30 minutes
Ratio of *Indigo Naturalis*:Olive Oil (w/v): 1:10

### Amount of INDIRUBIN extracted: 4.66 mg/g (mg of INDIRUBIN/g of Indigo Naturalis)

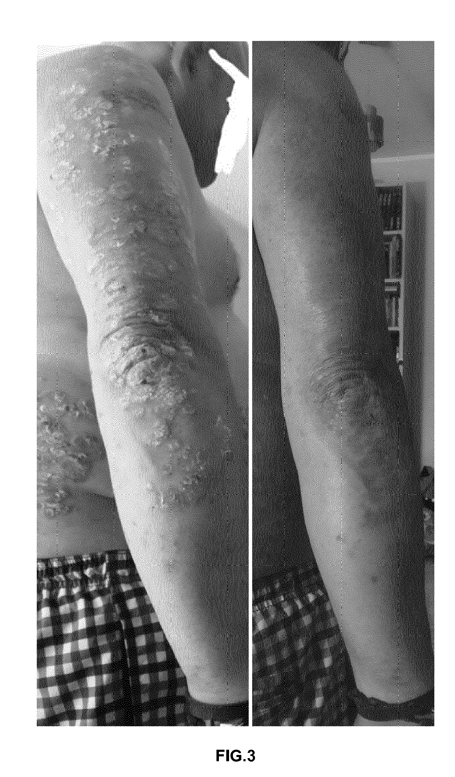

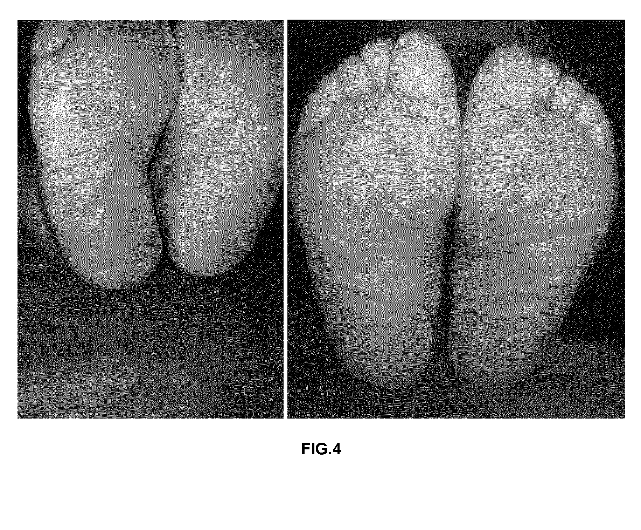

The photos of Figures 3 and 4 show the results obtained with the composition of the present invention, whereas the same patients treated with the composition described in EP2489358 did not give the same excellent results. More specifically, the composition according to the present invention, even when used at the same indirubin dosage of the extracts described in EP2489358, proves surprisingly more effective with respect to the phytocomplex obtainable as described in EP2489358.

Comparative experiments were also conducted on a group of 5 patients with a severe or moderate form of dermatitis of inflammatory origin, probably caused by contact with an external agent. Patients were treated in parallel with the olive oil extract obtained as described in EP2489358 and with the composition of the present invention, which uses isolated indirubin. Indirubin dosage being equal, only the zones and patients treated with the composition which uses isolated indirubin effectively cured dermatitis.

## Claims

1. A composition for topical use in the treatment of psoriasis or of dermatitides caused by inflammatory processes, comprising as active principle isolated Indirubin.

2. The composition according to claim 1 wherein with the exception of Indirubin the composition does not comprise other substances present in the extracts of leaves and/or pigments of the plants *Indigofera tinctoria, Baphieacanthuscusia, Isatistinctoria, Polygonum tinctorium, Isatis indigotica.*

3. The composition according to claim 1 or 2 for topical use in the treatment of plaque psoriasis, guttate psoriasis, inverse psoriasis and/or erythrodermic psoriasis.

4. The composition according to any one of claims 1 to 3 wherein the Indirubin is in a concentration between 0.1 and 10 mg per gram of composition.

5. The composition according to any one of claims 1 to 4 wherein the Indirubin is in a concentration between 1.00 and 10.00 mg per gram of composition.

6. The composition according to any one of claims 1 to 5 wherein the Indirubin is in a concentration between 5.00 and 10.00 mg per gram of composition.

7. The composition according to any one of claims 1 to 6 further comprising one or more perfuming agents and/or emulsifiers.

8. The composition according to any one of claims 1 to 7 wherein the Indirubin is isolated and extracted from the leaves and/or pigments of a plant selected from *Indigofera tinctoria, Baphieacanthuscusia, Isatistinctoria, Polygonum tinctorium, Isatis indigotica.*

9. The composition according to any one of claims 1 to 8 in the form of gel, ointment, cream, pomade, foam, powder, spray, oil-in-water emulsion, water-in-oil emulsion, oil-in-gel emulsion, gel-in-oil emulsion, suspension.

10. The composition according to any one of claims 1 to 9 for topical use on the scalp.

11. The composition according to any one of claims 1 to 10 wherein said composition is administered topically at least once a day for at least 10 days.

12. The composition according to any one of claims 1 to 10, further comprising Coco-Caprilate and/or Castor oil and/or Vaseline and/or Beeswax and/or Capric Triglyceride.

13. The composition according to any one of claims 1 to 12 wherein said Indirubin is not isolated by hot extraction in olive oil.

14. The composition according to any one of claims 1 to 13 wherein said dermatitis is a contact dermatitis.

15. A dressing for dermatological use comprising a composition according to any one of claims 1 to 14 in the form of gauze, patch or bandage.
